# FASCICULE DE BREVET EUROPEEN

(11) **EP 3 864 666 B1**
(45) Date de publication et mention de la délivrance du brevet: **01.04.2026**
(21) Numéro de dépôt: 18810971.4
(22) Date de dépôt: 23.11.2018
(51) Int. Cl.: G16H 40/63, G16H 20/13, G16H 20/17, G16H 20/10

(54) **TERMINAL MOBILE DE CONTRÔLE D'UN CONTENEUR D'UN PRODUIT MÉDICAL, ENSEMBLE, INSTALLATION ET PROCÉDÉ DE CONTRÔLE ASSOCIÉS**
MOBILES ENDGERÄT ZUM STEUERN EINES BEHÄLTERS EINES MEDIZINISCHEN PRODUKTES, ZUSAMMENBAU, INSTALLATION UND ZUGEHÖRIGES STEUERVERFAHREN
MOBILE TERMINAL FOR CONTROLLING A CONTAINER OF A MEDICAL PRODUCT, ASSEMBLY, INSTALLATION AND ASSOCIATED CONTROL METHOD

(30) Priorité: 12.10.2018 FR 1859494
(43) Date de publication de la demande: 18.08.2021
(73) Titulaire: Biolog-id, 92100 Boulogne-Billancourt (FR)
(72) Inventeur: MONGRENIER, Jean-Claude, 78000 Versailles (FR)
(74) Mandataire: Lavoix
(86) Numéro de dépôt international: PCT/EP2018/082451
(87) Numéro de publication internationale: WO 2020/074111

(56) Documents cités:
- EP-A1- 1 926 034
- EP-A1- 1 944 709
- WO-A1-2018/083399
- US-A1- 2010 072 280

## Description

### DOMAINE TECHNIQUE DE L'INVENTION

La présente invention concerne un terminal mobile. L'invention se rapporte également à un ensemble et une installation de contrôle comportant un tel terminal mobile et un procédé de contrôle associé.

### ARRIERE-PLAN TECHNOLOGIQUE

La présente invention se rapporte au domaine médical, plus spécifiquement au domaine de la chimiothérapie.

La chimiothérapie est l'usage de certaines substances chimiques pour traiter une maladie. C'est une technique de traitement à part entière au même titre que la chirurgie ou la radiothérapie.

Le terme « chimiothérapie » est principalement utilisé pour désigner les traitements médicamenteux (agents chimiothérapeutiques cytostatiques et antinéoplasiques) contre le cancer. On désigne l'antibiothérapie comme chimiothérapie antibactérienne mais, dans la pratique médicale, le mot est plus habituellement utilisé dans le contexte du traitement de la tuberculose.

Une autre utilisation des agents chimiothérapeutiques est le traitement des maladies auto-immunes.

Comme dans tout traitement, la chimiothérapie dépend beaucoup du patient considéré. L'administration à un patient d'une préparation de chimiothérapie destinée à un autre patient peut donc avoir des conséquences graves pour la santé du patient.

Or, dans les hôpitaux, il est observé des erreurs, ces erreurs nuisant d'une part à la santé du patient mais également à la réputation de l'hôpital.

Il est connu des techniques permettant de réduire ces erreurs des documents US 2010/072280 A1, EP 1 944 709 A1, WO 2018/083399 A1 et EP 1 926 034 A1.

### RESUME DE L'INVENTION

Il existe donc un besoin pour un appareil permettant, avec une mise en œuvre aisée, une augmentation de la capacité à contrôler les produits administrés à un patient avec une meilleure fiabilité.

A cet effet, la présente description porte sur un terminal mobile de contrôle selon la revendication 1.

Suivant des modes de réalisation particuliers, le terminal mobile présente la caractéristique de la revendication 2, 3 ou 4.

La présente description propose également un ensemble de contrôle d'au moins un conteneur selon la revendication 5.

Il est aussi proposé une installation de contrôle de conteneurs selon la revendication 6.

Suivant un mode de réalisation particulier, l'installation présente une ou plusieurs des caractéristiques des revendications 7 et 8, prise(s) isolément ou selon toutes les combinaisons techniquement possibles.

La présente description porte aussi sur un produit programme d'ordinateur selon la revendication 9.

La description concerne aussi un support lisible d'informations selon la revendication 10.

### BREVE DESCRIPTION DES DESSINS

Des caractéristiques et avantages de l'invention apparaîtront à la lecture de la description qui va suivre, donnée uniquement à titre d'exemple non limitatif, et faite en référence aux dessins annexés, sur lesquels :
- la figure 1 est une vue schématique d'une infrastructure médicale ;
- la figure 2 est une vue schématique d'une première partie de l'infrastructure médicale de la figure 1 ;
- la figure 3 est une représentation schématique d'un appareil de la première partie de l'infrastructure médicale de la figure 2 ;
- la figure 4 est une représentation schématique d'un dispenseur ;
- la figure 5 est une vue en perspective d'un exemple du dispenseur ;
- la figure 6 est une en perspective du dispenseur de la figure 5 sans les parois externes ;
- la figure 7 est une vue schématique d'une antenne latérale faisant partie du dispenseur de la figure 5 ;

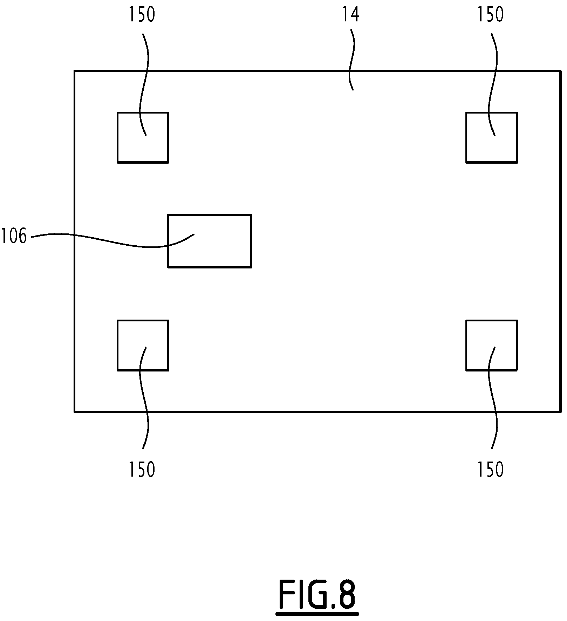

- la figure 8 est une vue schématique d'une deuxième partie de l'infrastructure médicale de la figure 1 ;
- la figure 9 est une représentation schématique d'une borne faisant partie de la deuxième partie de la figure 8 ;
- la figure 10 est une vue schématique d'une troisième partie de l'infrastructure médicale de la figure 1 ;
- la figure 11 est une vue schématique d'une quatrième partie de l'infrastructure médicale de la figure 1 ;
- la figure 12 est une représentation d'un terminal mobile en fonctionnement dans la quatrième partie de l'infrastructure médicale, et
- les figures 13 à 16 sont des exemples de menus affichés par le terminal mobile.

### DESCRIPTION DETAILLE DE MODES DE REALISATION PARTICULIERS

### Description générale d'une installation de contrôle destinée à une infrastructure

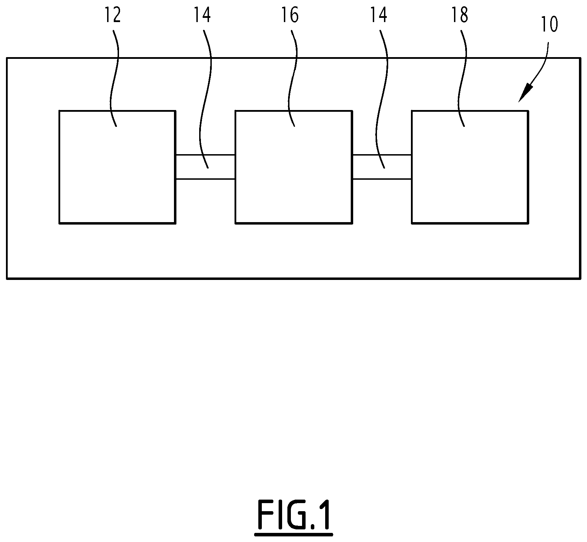

Une infrastructure 10 est représentée schématiquement sur la figure 1.

L'infrastructure 10 est une infrastructure médicale.

Dans l'exemple proposé, l'infrastructure 10 est un ensemble hospitalier.

Selon d'autres modes de réalisation, l'infrastructure 10 est un centre de soin ou une clinique.

En variante, l'infrastructure 10 est un ensemble de lieux permettant de mettre en œuvre une hospitalisation à domicile.

Plus généralement, l'infrastructure 10 est un ensemble de lieux permettant la mise en œuvre d'un ou plusieurs traitements médicaux sur au moins un patient.

Dans le cas de la figure 1, l'infrastructure 10 comporte un centre de préparation 12, un espace de transport 14, un poste de soin 16 et une chambre 18.

L'infrastructure 10 est munie d'une installation de contrôle.

L'installation de contrôle permet de contrôler au moins un conteneur comprenant un produit médical destiné à un patient unique.

Le produit médical est, par exemple, un médicament à administrer à un patient.

Le médicament est une préparation magistrale, réalisée pour un patient précis, notamment à la suite d'une ordonnance nominative.

Le médicament est administré au patient par injection.

Le médicament est, par exemple, un composé utilisé en chimiothérapie.

En variante, le produit médical est un agent de contraste.

Un agent de contraste est un agent augmentant artificiellement le contraste afin de mieux visualiser par imagerie médicale une structure anatomique telle un organe ou pathologique telle une tumeur.

En variante, le produit médical est une poche contenant des produits biologiques, tels que des produits sanguins (poche de sang primaire, de plasma, de plaquettes, de globules rouges...) ou des produits d'ingénieries cellulaires (cellules, souches...).

En variante, le produit médical est un dispositif médical implantable (DMI).

Le produit médical est, par exemple, une prothèse à poser chez un patient, un implant d'une partie du corps ou une endoprothèse telle un stent.

Le produit médical est, par exemple, un dispositif médical implantable actif (DMIA).

Le produit médical est, par exemple, un stimulateur d'une partie du corps comme un stimulateur cardiaque, une sonde ou une pompe.

L'installation de contrôle s'appuie en particulier sur la technologie RFID (acronyme de « Radio Frequency Identification » signifiant littéralement « identification radiofréquence »).

L'installation de contrôle associe une étiquette électronique (tag RFID) apposée sur chaque préparation de chimiothérapie des équipements de lecture et d'encodage de tag placés au point stratégique du circuit et un logiciel articulé en quatre modules.

L'étiquette électronique 108 est une étiquette électronique de communication sans fil.

Selon l'exemple décrit, l'étiquette électronique 108 est une puce RFID (de l'anglais « radio frequency identification » signifiant en français « identification radiofréquence »).

Pour donner un ordre de grandeur, la puce RFID est un rectangle de 35 millimètres (mm) de longueur par 20 mm de largeur. Néanmoins, la puce RFID n'est pas limitée à cette géométrie et peut présenter des dimensions et des formes variables (carré, rectangulaire, rond...).

L'installation comprend une pluralité d'ensembles destinés à assurer la traçabilité de conteneurs de préparations de chimiothérapie depuis la préparation jusqu'à l'administration à un patient ayant besoin de ladite préparation de chimiothérapie.

Les ensembles de l'installation sont destinés à être utilisés en combinaison avec des logiciels commerciaux ou des lecteurs de code-barres.

Chacun des ensembles est présenté successivement dans ce qui suit en référence à un lieu de l'infrastructure 10.

Quatre ensembles vont ainsi être décrits pour le cas de la figure 1 : un premier ensemble pour le centre de préparation 12, un deuxième ensemble pour l'espace de transport 14, un troisième ensemble pour le poste de soin 16 et un quatrième ensemble pour la chambre 18.

### Description d'un premier ensemble de l'installation

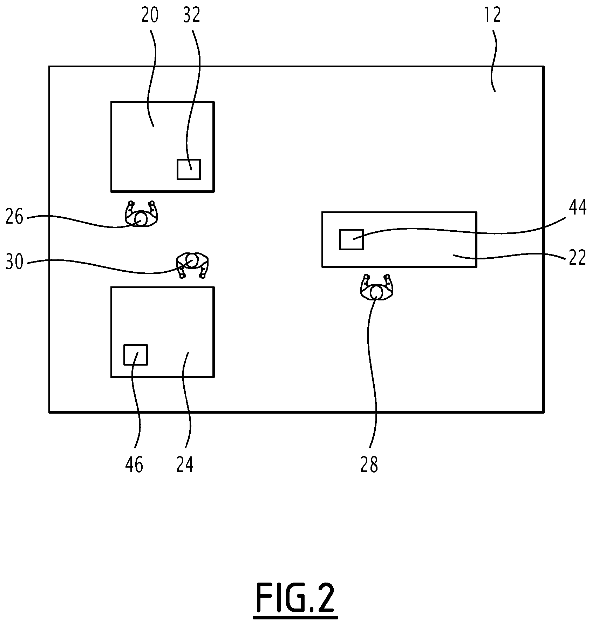

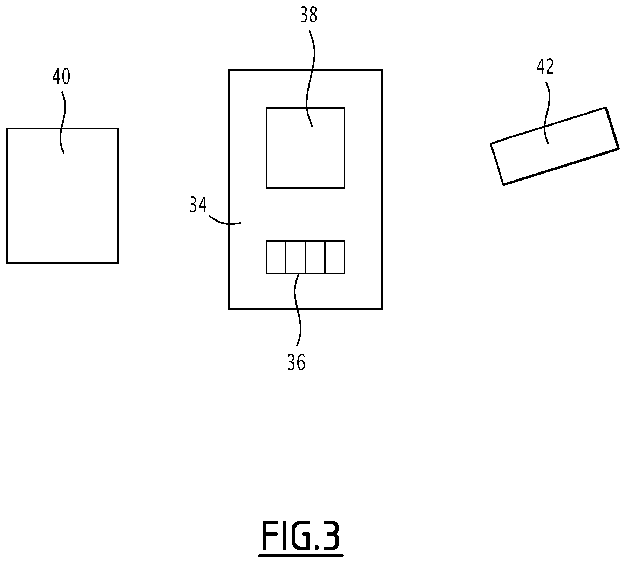

Pour décrire le premier ensemble, il est décrit le centre de préparation 12 en référence aux figures 2 et 3.

Le centre de préparation 12 comporte trois postes 20, 22 et 24 utilisés par un opérateur respectif 26, 28 et 30.

Le premier poste 20 est un poste d'initialisation 20.

Dans le poste d'initialisation 20, le premier opérateur 22 effectue une série d'intervention à l'aide d'une première station de travail 32 destiné à initialiser un conteneur 34.

Le conteneur 34 est un conteneur de préparations de chimiothérapie.

A titre d'exemple, le conteneur est une poche ou une seringue.

Comme visible sur la figure 3, le conteneur 34 est pourvu d'un code-barres 36 et tag 38.

Le tag 38 a été obtenu par une imprimante qui fait, selon un mode de réalisation particulier, partie de la première station de travail 32.

Pour interagir avec le conteneur 34, la première station de travail 32 est pourvue d'un ordinateur 40 et d'un lecteur 42.

Le tag 38 est un tag RFID.

L'ordinateur 40 et un produit programme d'ordinateur sont propres à interagir pour mettre en œuvre un procédé de contrôle d'un ou plusieurs conteneur(s).

Plus généralement, l'ordinateur 40 est un calculateur électronique propre à manipuler et/ou transformer des données représentées comme des quantités électroniques ou physiques dans des registres du calculateur et/ou des mémoires en d'autres données similaires correspondant à des données physiques dans des mémoires, des registres ou d'autres types de dispositifs d'affichage, de transmission ou de mémorisation.

L'ordinateur 40 comporte un processeur comprenant une unité de traitement de données, des mémoires et un lecteur de support d'informations. L'ordinateur 40 comprend également un clavier et une unité d'affichage.

Le produit programme d'ordinateur comporte un support lisible d'informations.

Un support lisible d'informations est un support lisible par l'ordinateur 40, usuellement par le lecteur. Le support lisible d'informations est un médium adapté à mémoriser des instructions électroniques et capable d'être couplé à un bus d'un système informatique.

A titre d'exemple, le support lisible d'informations est une disquette ou disque souple (de la dénomination anglaise de « *floppy disk* »), un disque optique, un CD-ROM, un disque magnéto-optique, une mémoire ROM, une mémoire RAM, une mémoire EPROM, une mémoire EEPROM, une carte magnétique ou une carte optique.

Sur le support lisible d'informations est mémorisé un premier programme d'ordinateur comprenant des instructions de programme. Le premier programme d'ordinateur constitue le premier module des quatre modules précités.

Le programme d'ordinateur est chargeable sur l'unité de traitement de données et est adapté pour entraîner la mise en œuvre du procédé de contrôle.

Le lecteur 42 est un propre à lire des informations sur le tag 38 ainsi qu'à écrire des informations sur le tag 38.

En ce sens, le lecteur 42 est un lecteur RFID.

Le deuxième poste 22 est un poste de préparation 22.

Le poste de préparation 22 est pourvu d'un appareil de fabrication de médicaments 44.

Par exemple, l'appareil de fabrication 44 comporte une partie stérile à laquelle le deuxième opérateur 28 a accès par l'emploi de gants.

Le troisième poste 24 est un poste d'envoi 24.

Le poste de préparation 24 est pourvu d'une deuxième station mobile 46.

En fonctionnement, au premier poste 20, la première station de travail 32 permet à un premier opérateur 26 de se connecter en scannant un badge sur un lecteur. L'écran de l'ordinateur affiche le compte de l'opérateur.

Chaque conteneur 34 est préparé et équipé d'un tag RFID est placé sur un lecteur et scanné pour s'assurer que le tag est vide.

L'opérateur est ensuite invité à scanner le code-barres du conteneur 34.

Le conteneur 34 est ensuite utilisé au deuxième poste 22 par un deuxième opérateur 28 pour mettre la préparation de chimiothérapie à l'intérieur du conteneur 34.

Un troisième opérateur 30 écrit dans la mémoire du tag RFID le nom de la personne qui a préparé le conteneur, la préparation contenue et le nom du patient associé.

Le statut « préparé » est attribué au conteneur 34.

De manière générale, de multiples informations peuvent être présentes sur le tag, notamment le numéro d'ordonnancier du produit, la dénomination commune internationale du produit, la dose du produit, la date d'expiration du produit, la voie d'administration du produit ou le service de destination du produit.

Le premier ensemble permet ainsi d'obtenir un conteneur 34 dans l'état préparé.

### Description d'un deuxième ensemble de l'installation

### Description d'un dispenseur

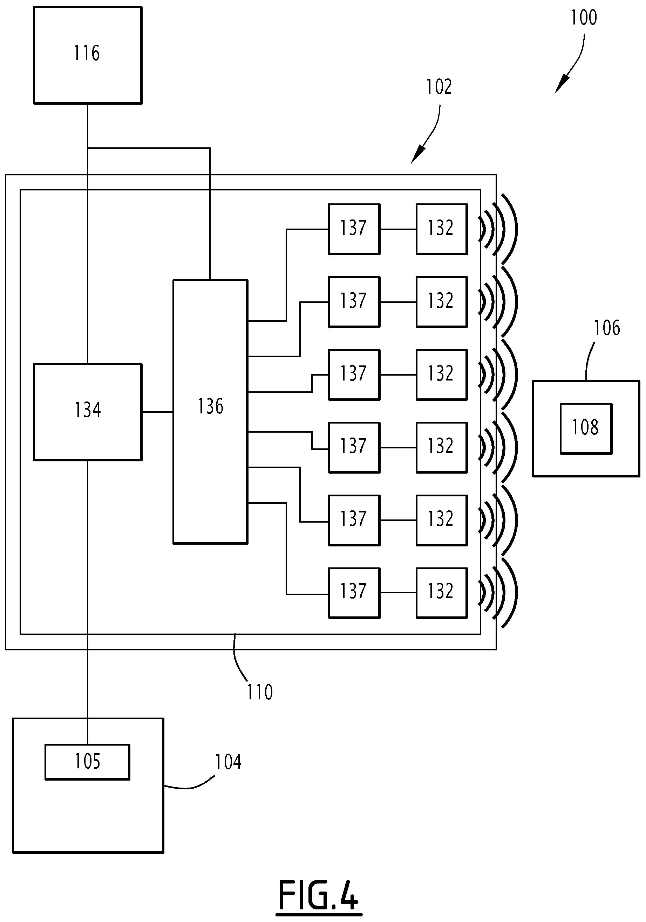

Un exemple de dispenseur 100 est représenté sur la figure 4.

Le dispenseur 100 comprend au moins une partie de réception 102 et au moins un contrôleur 104.

Le contrôleur 104 est adapté à communiquer avec l'au moins une partie de réception 102. La partie de réception 102 est également propre à communiquer avec le contrôleur 104.

Le contrôleur 104 et la partie de réception 102 communiquent selon une liaison sans fil.

Par exemple, le contrôleur 104 et la partie de réception 102 communiquent au moyen du protocole de réseau local à commutation de paquets, tel un protocole Ethernet.

Le contrôleur 104 comprend une interface homme-machine 105 propre à commander la partie de réception 102 et à afficher au moins une information lue par la partie de réception 102.

L'interface homme-machine 105 comprend, par exemple, un écran.

Selon un mode de réalisation particulier, l'écran est tactile de sorte qu'un opérateur est apte à interagir avec la partie de réception 102, par exemple en validant les informations affichées. L'écran tactile est compatible avec des gants.

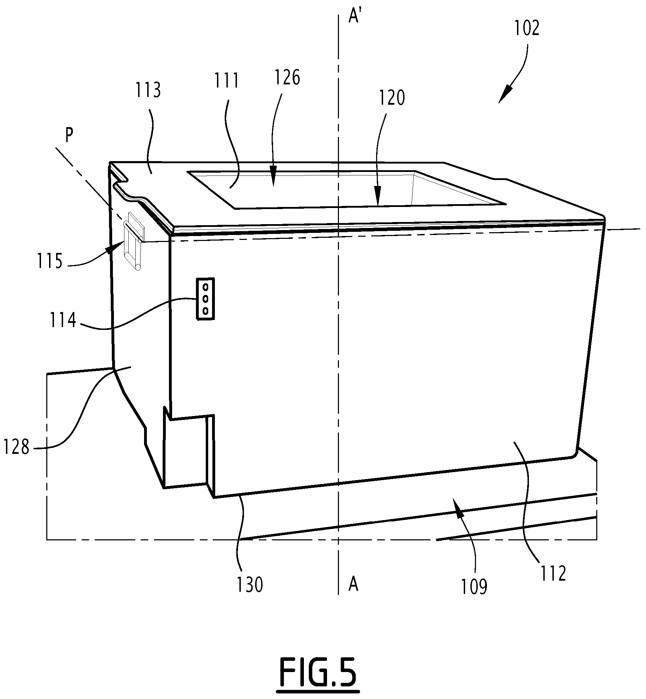

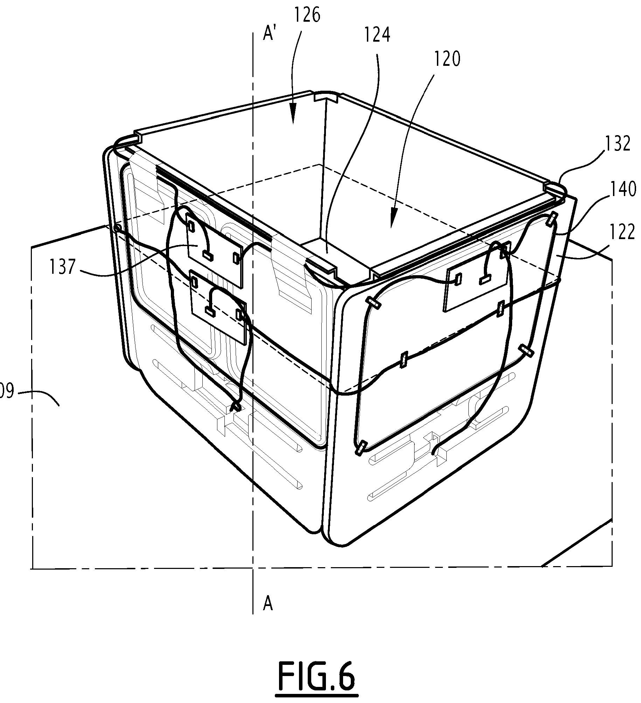

Les figures 5 et 6 présentent des exemples de vues partiels ou totales de la partie de réception 102.

Le dispenseur 100 est configuré pour contrôler au moins un conteneur 106.

La partie de réception 102 est propre à recevoir l'au moins un conteneur 106 ou un colis comprenant l'au moins un conteneur 106.

La partie de réception 102 est propre à reposer sur une surface de support 109 comme une table par exemple.

Dans ce qui suit, les termes « vertical » et « horizontal » s'entendent par rapport à la surface de support 109. En particulier, par « vertical », il est entendu perpendiculaire à la surface de support 109.

La partie de réception 102 comprend un lecteur 110 et une pluralité de parois.

La pluralité de parois comprend une pluralité de parois internes 111, une pluralité de parois externes 112 et des parois de liaison 113.

La partie de réception 102 comprend, dans l'exemple de la figure 5, en outre, un dispositif indicateur 114 et une paire de poignées 115 permettant le déplacement du dispenseur 102.

La partie de réception 102 est alimentée électriquement par le secteur 116.

Dans l'exemple de la figure 5, la partie de réception 102 comprend cinq parois internes 111 et cinq parois externes 112.

Les parois internes 111 sont réalisées en plastique, par exemple en polyéthylène ou en polypropylène.

Les parois internes 111 délimitent un volume de stockage 120.

Le volume de stockage 120 est, dans l'exemple de la figure 5, de forme parallélépipédique.

La partie de réception 102 est propre à recevoir l'au moins un conteneur 106 ou un colis comprenant l'au moins un conteneur 106 dans le volume stockage 120.

Le volume de stockage 120 présente un volume compris entre 0,015 mètres cube (m³) et 0,03 m³, par exemple 0,022 m³.

Le rapport entre le volume de stockage 120 et le volume de la partie de réception 102 est compris entre 20 % et 30 %, par exemple 25 %.

Les parois internes 111 comprennent des parois latérales internes 122, ici quatre parois latérales internes 122, et une paroi inférieure interne 124 sur laquelle les conteneurs 106 ou le colis comprenant les conteneurs 106 sont destinés à reposer.

La paroi inférieure interne 124 s'étend dans un plan horizontal.

La hauteur de la paroi inférieure interne 124 par rapport à la surface de support 109 de la partie de réception 102, est comprise entre 50 mm et 150 mm, par exemple 117 mm.

Chaque paroi latérale interne 122 s'étend dans un plan vertical, perpendiculairement depuis un côté de la paroi inférieure interne 124.

Chaque paroi latérale interne 122 présente une hauteur selon l'axe vertical A-A' comprise entre 200 mm et 300 mm, par exemple 218 mm.

Chaque paroi latérale interne 122 présente une longueur selon une direction horizontale comprise entre 300 mm et 500 mm, par exemple 282 mm ou 355 mm.

Comme visible sur la figure 6, les parois latérales internes 122 délimitent une unique ouverture d'accès 126 au volume de stockage 120.

La partie de réception 102 ne comprend donc pas d'ouverture d'accès au volume de stockage 120 traversante.

L'ouverture d'accès 126 définit un plan d'ouverture P.

Le plan d'ouverture P est un plan horizontal, perpendiculaire à l'axe A-A'.

Les parois externes 112 forment la structure extérieure de la partie de réception 102.

Comme visible sur la figure 5, la partie de réception 102 est une structure de forme parallélépipédique.

Chaque paroi externe 112 est réalisée en un matériau électriquement conducteur présentant une conductivité électrique supérieure ou égale à 0,3 méga Siemens par cm (MS.cm⁻¹).

Par exemple, chaque paroi externe 112 est réalisée en aluminium.

Les parois externes 112 jouent ainsi le rôle d'isolation électromagnétique de l'intérieur de la partie de réception 102.

Les parois externes 112 délimitent un volume interne. Le volume interne est égal au volume de la partie de réception 102.

La partie de réception 102 présente un volume inférieur à 0,1 m³, par exemple 0,095 m³.

Le volume de stockage 120 est disposé dans le volume interne.

Les parois externes 112 comprennent des parois latérales externes 128, ici quatre parois latérales externes 128, et une paroi inférieure externe 130.

La partie de réception 102 est propre à reposer sur la surface de contact 109 via la paroi inférieure externe 130.

La paroi inférieure externe 130 s'étend dans un plan horizontal.

Chaque paroi latérale externe 128 s'étend dans un plan vertical, perpendiculairement depuis un côté de la paroi inférieure externe 130.

Chaque paroi latérale externe 128 présente une hauteur selon l'axe vertical A-A' comprise entre 300 mm et 400 mm, par exemple 335 mm.

Chaque paroi latérale externe 128 présente une longueur selon une direction horizontale comprise entre 400 mm et 600 mm, par exemple 480 mm ou 588 mm.

Dans l'exemple de la figure 5, deux des parois latérales externes 130 sont parallèles à deux des parois latérales internes 122. Les deux autres parois latérales externes 130 sont parallèles aux deux autres parois latérales internes 122.

Les parois de liaison 113 relient les extrémités libres des parois latérales internes 122 et des parois latérales externes 130.

Le lecteur 110 est propre à lire l'au moins une information mémorisée dans la mémoire de chaque étiquette électronique 108.

En particulier, le lecteur 110 est propre à lire simultanément l'au moins une information mémorisée dans la mémoire d'au moins vingt étiquettes électroniques 108.

Par « simultanément », il entendu une lecture suite à la même impulsion électromagnétique émise par le lecteur 110.

Le lecteur 110 est propre à fonctionner selon un protocole de communication adapté pour lire l'au moins une information mémorisée dans la mémoire de chaque étiquette électronique 108. Le protocole de communication est, par exemple, un protocole de communication RFID.

Le lecteur 110 est apte à émettre ou à recevoir un signal ayant une fréquence comprise entre 13,540 méga Hertz (MHz) et 13,567 MHz.

Le lecteur 110 comprend une pluralité d'antennes 132.

Le lecteur 110 comprend, en outre, au moins un module de lecture 134 et un multiplexeur 136.

Le multiplexeur 136 relie les antennes 132 au module de lecture 134.

Le module de lecture 134 est configuré pour lire l'au moins une information mémorisée dans la mémoire de chaque étiquette électronique 108 et pour envoyer un signal comprenant l'au moins une information au contrôleur extérieur 104 à la partie de réception 102.

Le lecteur 110 comprend, en outre, pour chaque antenne 132, un adaptateur d'antenne 137 disposé entre l'antenne 132 et le multiplexeur 136.

L'adaptateur d'antenne 137 permet d'optimiser le transfert de puissance entre une antenne 132 et le reste du lecteur 110 en modulant l'impédance de celui-ci.

L'adaptateur d'antenne 137 est ici réglable manuellement au moyen d'une molette par exemple.

Chaque antenne 132 est sous forme de boucle.

Chaque antenne 132 définit une surface interne. La surface interne de chaque antenne 132 est supérieure ou égal à 0,04 m².

Chaque antenne 132 est propre à créer un champ magnétique orienté selon une direction.

Chaque antenne 132 est un câble coaxial. Le câble coaxial est un câble à deux conducteurs de pôles opposés séparés par un isolant.

Par exemple, le câble coaxial est du type RG58, bien connu de l'homme du métier.

Chaque paroi latérale interne 122 comporte un support 138 propre à loger au moins une partie de l'une des antennes 132.

Le support 138 est par exemple une bride vissée sur la paroi latérale interne 122.

Le support 138 définit avec la paroi latérale interne 122 une section de passage pour l'antenne 132.

En particulier, chaque paroi latérale interne 122 reçoit au moins une antenne 132, dite antenne latérale 140.

Chaque antenne latérale 140 est fixée sur la surface extérieure de la paroi latérale interne 122 associée.

Le rapport entre la surface interne de chaque antenne latérale 140 et la surface de la paroi latérale interne 122 associée est supérieur ou égal à 75 %.

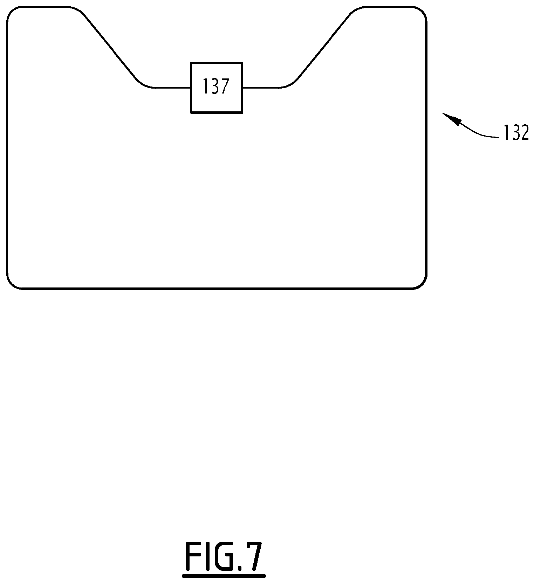

Comme visible sur la figure 7, chaque antenne latérale 140 présente, par exemple, une forme de rectangle à coins arrondis.

L'un des côtés du rectangle forme une sinusoïde vers l'intérieur du rectangle, l'antenne latérale 140 étant reliée à son adaptateur d'antenne 137 respectif au niveau de la sinusoïde.

En particulier, le dit côté comprend cinq intervalles.

Le premier intervalle s'étend depuis un coin du rectangle.

Le premier intervalle est perpendiculaire au côté adjacent du rectangle.

Le deuxième intervalle s'étend depuis le premier intervalle. Le premier intervalle et le deuxième intervalle sont reliés par un premier raccord arrondi.

Le deuxième intervalle forme avec le premier intervalle un angle compris entre 30 degrés (°) et 60 °.

Le troisième intervalle s'étend depuis le deuxième intervalle. Le deuxième intervalle et le troisième intervalle sont reliés par un deuxième raccord arrondi.

Le troisième intervalle est parallèle au premier intervalle.

Le troisième intervalle est relié à l'adaptateur d'antenne 137 associé.

Le quatrième intervalle s'étend depuis le troisième intervalle. Le troisième intervalle et le quatrième intervalle sont reliés par un troisième raccord arrondi.

Le quatrième intervalle forme avec le troisième intervalle un angle compris entre 30 ° et 60 °.

Le cinquième intervalle s'étend depuis le quatrième intervalle. Le cinquième intervalle et le quatrième intervalle sont reliés par un quatrième raccord arrondi.

Le cinquième intervalle est parallèle au troisième intervalle et s'étend dans le prolongement du premier intervalle.

Le cinquième intervalle est relié à un coin du rectangle.

Le cinquième intervalle est perpendiculaire au côté adjacent du rectangle.

Au moins une antenne 132 est agencée sur l'ensemble des parois latérales internes 122 et s'étend dans le plan d'ouverture P. Ladite antenne 132 est agencée sur le pourtour de l'ouverture d'accès 126.

Au moins une antenne 132 est agencée sur l'ensemble des parois latérales à l'écart du plan d'ouverture P. Ladite antenne 132 s'étend, par exemple, dans un plan parallèle au plan d'ouverture P. Ladite antenne 132 s'étend par exemple dans un plan horizontal de hauteur, par rapport à la surface de support 109, comprise entre 200 mm et 250 mm, par exemple 223 mm.

Le dispositif indicateur 114 est propre à émettre un signal lorsque la partie de réception 102 est branché à une source d'alimentation électrique.

Le dispositif indicateur 114 est, en outre, propre à émettre un signal lorsque le lecteur 110 est en cours de lecture d'au moins une étiquette électronique 108.

Le dispositif indicateur 114 est, en outre, propre à émettre un signal en fonction de l'information lue sur chaque étiquette électronique 108.

Dans un mode de réalisation particulier, le dispositif indicateur 114 est un dispositif lumineux. Par exemple, le dispositif indicateur 114 comprend trois lampes LED de couleurs différentes.

Chaque poignée 115 est fixée sur une paroi latérale externe 128 au moyen de vis par exemple.

Chaque poignée 115 est propre à être saisie par la main d'un opérateur désirant transporter la partie de réception 102. Les deux poignées 115 sont disposées sur deux faces latérales externes 128 parallèles.

Le fonctionnement du dispenseur 100 est maintenant expliqué en référence à un exemple de mise en œuvre d'un procédé de contrôle d'au moins un conteneur 106 comprenant un produit médical au moyen d'un système de contrôle 100.

Une pluralité de conteneurs 106 est à vérifier.

Par exemple, vingt conteneurs 106 sont à vérifier.

La pluralité de conteneurs 106 est placée dans le volume de stockage 120 de la partie de réception 102 par un opérateur.

En variante, la pluralité de conteneurs 106 est placée dans un colis et le colis est placé dans le volume de stockage 120 par l'opérateur.

L'opérateur utilise l'interface homme-machine 105 pour demander au lecteur 110 de lire les étiquettes électroniques 108 présentes dans la partie de réception 102.

Le procédé de contrôle comprend alors une étape de lecture par le lecteur 110 de l'au moins une information mémorisée sur la mémoire de chaque étiquette électronique 108.

En particulier, la lecture est effectuée par le module de lecture 134 à partir des signaux envoyés par les six antennes 132 via le multiplexeur 136.

Le module de lecture 134 envoie alors un signal comprenant l'au moins une information au contrôleur 104.

L'au moins une information est affichée sur l'interface homme-machine 105.

Dans l'hypothèse où les informations sont validées par l'opérateur ou par le contrôleur 104, les conteneurs 106 sont considérés comme valides et le statut de chaque conteneur 106 est modifié de « préparé » à « dispensé ».

Les conteneurs 106 valides sont alors par exemple envoyés à un centre de soins pour traiter un patient.

Dans le cas contraire, les conteneurs 106 présentant une information non validée sont mis de côté par l'opérateur.

De multiples variantes du dispenseur 100 sont possibles.

Selon une variante, l'écran de l'interface homme-machine 105 présente un clavier ou des boutons.

Selon une autre variante, le dispenseur 100 est alimenté par une alimentation propre, telle qu'une batterie par exemple.

Selon une autre variante, l'adaptateur d'antenne 137 est propre à se régler automatiquement.

Selon une autre variante, de multiples variantes de la forme des antennes 132 sont possibles en fonction de la nature des étiquettes électroniques 108. Par exemple, chaque antenne 132 est de forme circulaire ou carré.

Selon une autre variante, le dispositif indicateur 114 est un dispositif sonore.

### Description d'un exemple spécifique d'utilisation du dispenseur avec d'autres éléments

Le dispenseur 100 décrit précédemment a été proposé pour une utilisation seule.

Toutefois, le dispenseur 100 est avantageusement utilisé en combinaison avec d'autres éléments pour former le deuxième ensemble.

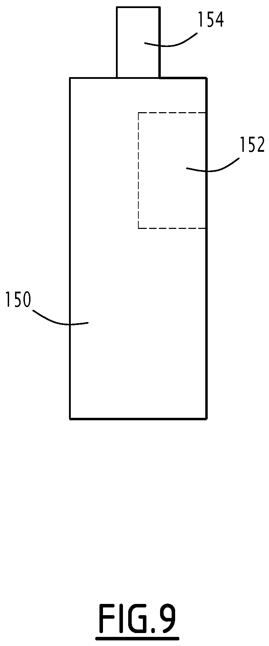

Comme visible sur la figure 9, le deuxième ensemble comporte un dispenseur 100 et quatre bornes de contrôle 150 pourvues d'un lecteur RFID.

Une borne de contrôle 150 est détaillée à la figure 9.

La borne de contrôle 150 comporte une antenne 152 et un dispositif d'avertissement 154.

L'antenne 152 permet de lire le tag 38.

L'écran d'affichage 154 permet d'avertir en cas de détection d'une anomalie.

Par exemple, si la borne de contrôle 150 est utilisé comme un ordinateur interagissant avec un module des quatre modules précités, le dispenseur 100 est connecté à la borne de contrôle 150. Une destination pour les conteneurs est entrée par l'utilisateur.

Une fois que les conteneurs destinés à être distribués à la destination entrée sont placés dans le dispenseur 100, tous les tags sont lus. La borne de contrôle 150 affiche pour chaque conteneur 34 sa destination et son statut. Si la destination est correcte, les données du conteneur 34 apparaissent en vert. Si la destination est incorrecte, les données du conteneur apparaissent en rouge et la dispensation de tous les conteneurs 34 est bloquée. L'utilisateur est alors invité à enlever le mauvais conteneur 34 ou les mauvais conteneurs 34 pour continuer la dispensation.

Le statut du conteneur 34 est modifié de « préparé » à « dispensé » en un clic.

Cela permet ainsi la dispensation groupée en lecture simultanée jusqu'à 20 produits.

L'installation permet d'informer en temps réel la logistique et les salles de soin.

L'installation permet également de gérer en toute sécurité les retours et les réattributions.

Les quatre modules précités sont mis à jour en temps réel, ce qui permet de suivre l'acheminement des préparations après dispensation.

La dispensation peut également avoir lieu au niveau du centre de préparation 12. Dans un tel cas, le deuxième ensemble vise à suivre le colis depuis l'envoi du centre de préparation 12 jusqu'au poste de soin 16.

Les statuts pour le conteneur 34 sont alors « enlevé » ou « livré ».

Un tel ensemble permet ainsi de suivre en temps réel les départs et les arrivées depuis le départ jusqu'à l'arrivée. Cela permet d'éviter toute erreur d'acheminement.

### Description d'un troisième ensemble de l'installation

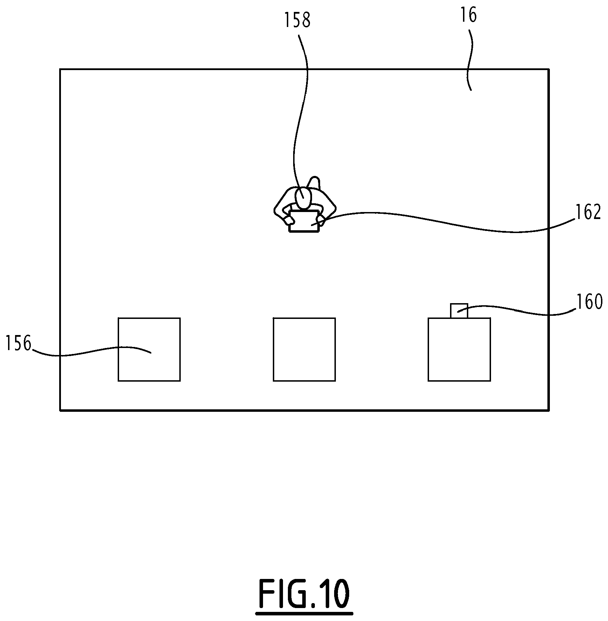

La figure 10 montre un poste de soin 16 équipé d'armoires de conservation 156 destinés à conserver les produits médicaux.

Un opérateur 158 porte une tablette 160 munie d'un lecteur RFID et interagissant avec un ordinateur central 162.

Le fonctionnement est similaire au fonctionnement précédent à savoir que l'écran de la tablette 160 affiche les données du conteneur en vert si le bon conteneur a atteint la destination correcte.

Lorsqu'un problème a lieu, par exemple que le patient n'a plus besoin de traitement, il est possible de renvoyer le conteneur 34 à une pharmacie et à l'attribuer à un patient différent.

Les nouveaux statuts sont « réceptionné », « non administré » et « à retourner ».

Cela permet de suivre le statut et l'acheminement des préparations en temps réel.

Cela rend possible l'automatisation du contrôle en réception.

L'installation offre la possibilité de programmer le retour d'une préparation.

### Description d'un quatrième ensemble de l'installation

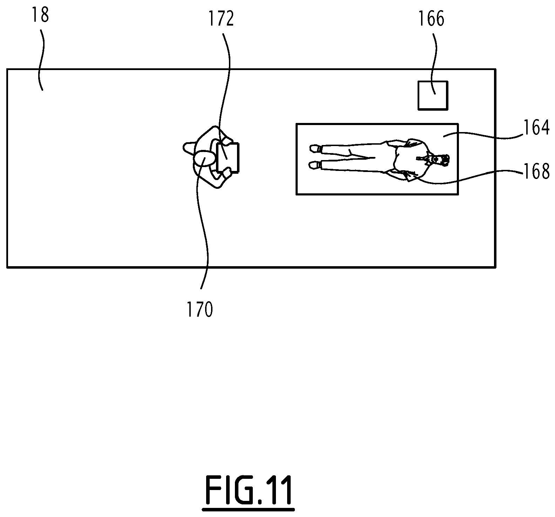

Pour décrire le quatrième ensemble, il est décrit la chambre 18 en référence à la figure 11.

La chambre 18 comporte un lit 164 et une colonne de délivrance de produits 166.

Un patient 168 est allongé sur le lit 164.

Un opérateur médical 170 est également présent. L'opérateur médical 170 cherche à administrer la préparation de chimiothérapie au patient 168.

L'opérateur médical 170 est, par exemple, une infirmière.

L'opérateur 170 détient un terminal mobile sous forme d'un assistant personnel 172.

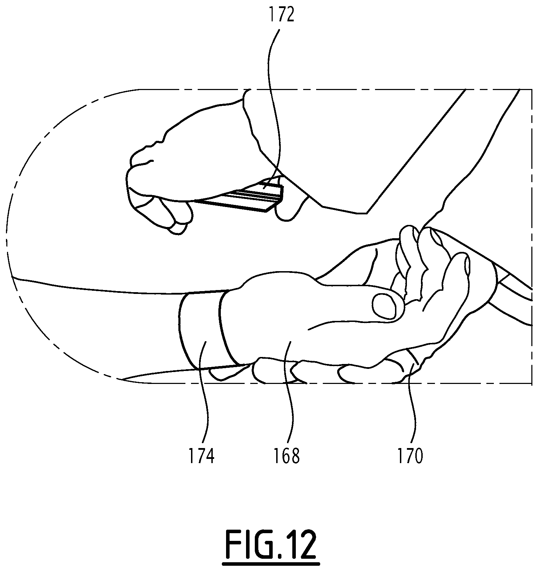

Comme visible sur la figure 12, le patient 168 est muni d'un bracelet d'identification 174.

Le bracelet d'identification 174 est pourvu d'un code d'identification propre au patient 168.

Le code d'identification est sous forme d'un code-barres et comporte des deuxièmes données 192.

L'assistant personnel 172 communique avec un serveur central selon un protocole de communication sans fil.

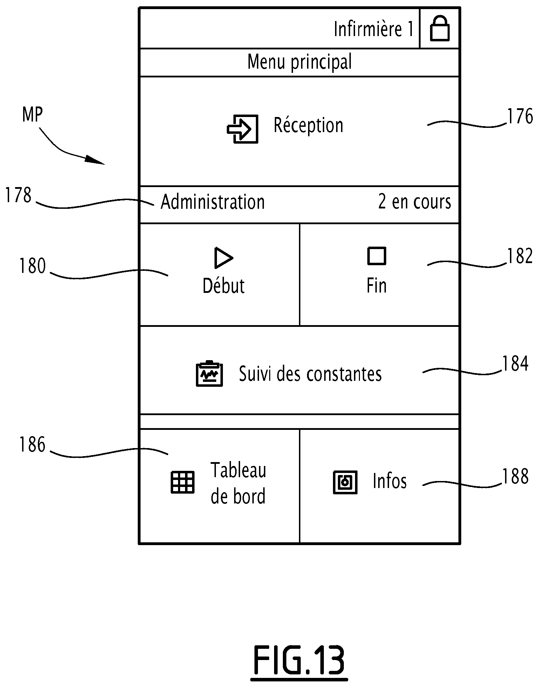

L'assistant personnel 172 est propre à rendre accessible à l'opérateur médical 170 une pluralité de fonctionnalités qui sont décrites en référence aux figures 13 à 16 montrant plusieurs menus.

L'assistant personnel 172 comporte un premier lecteur de code-barres lisant les deuxièmes données sur un bracelet d'identification propre à un patient, un deuxième lecteur lisant les premières données et un contrôleur comparant les premières données et les deuxièmes données pour obtenir un résultat de comparaison et validant que le produit médical est destiné au patient portant le bracelet d'identification en fonction du résultat de comparaison.

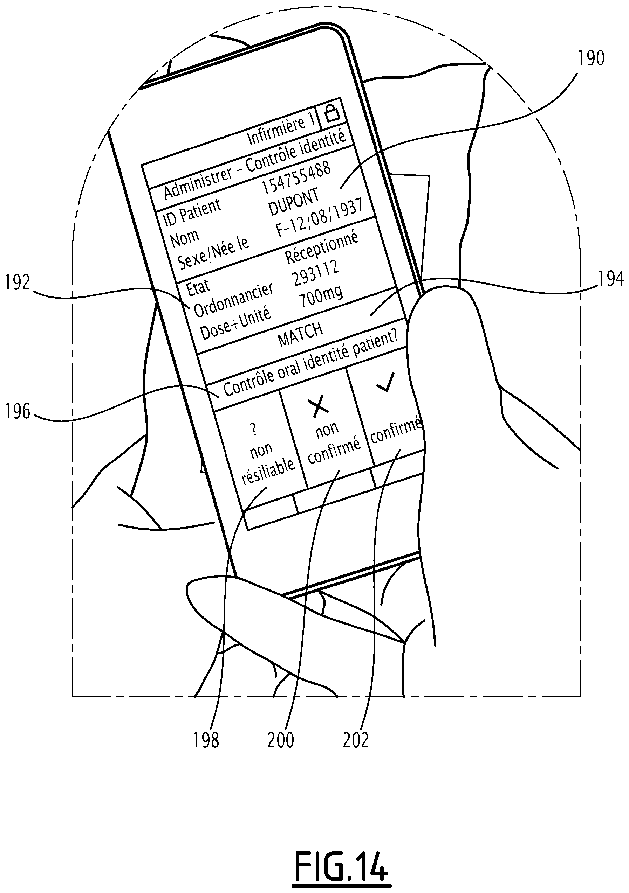

Cela est montré par un menu visible à la figure 14.

Dans ce menu sont affichées des premières données 190 et des deuxièmes données 192.

Les premières données 190 sont les données issues par le lecteur RFID du tag de la préparation de chimiothérapie.

Les deuxièmes données 192 sont les données issues par la lecture du code-barres du bracelet d'identification 174 du patient 168.

Le menu comporte, en outre, une pluralité de zones interactives : une zone 196 interrogeant l'opérateur 170 si le contrôle oral de l'identité du patient 168 a été effectué, un bouton 198 indiquant que le contrôle est non réalisable, un bouton 200 indiquant que l'identité du patient 168 est non confirmée et un bouton 202 indiquant que l'identité du patient 168 est confirmée.

L'assistant personnel 172 affiche « MATCH » dans la zone 194 de la figure 14 quand le bon conteneur 34 est attribué au bon patient. Sinon, l'assistant personnel 172 affiche « STOP » dans la zone 194. L'assistant personnel 172 affiche le temps restant pour l'administration. A la fin de l'administration ou durant l'administration si besoin, l'opérateur 170 scanne une fois encore le conteneur 34 pour arrêter l'administration.

Les nouveaux statuts sont « en cours d'administration » et « administré ».

De manière optionnelle, l'assistant personnel 172 peut proposer des fonctionnalités additionnelles.

Ces fonctionnalités apparaissent en regardant le menu principal MP présenté à la figure 13.

Le menu principal MP comporte une pluralité de zones interactives : une zone de réception 176, un affichage des administrations en cours 178, une action de déclenchement d'un début 180, une action de déclenchement d'une fin 182, un bouton d'accès au suivi des constantes 184, un bouton d'accès à un tableau de bord 186 et un bouton d'accès à des informations 188.

Une première fonctionnalité est de permettre d'enregistrer des constantes physiologiques.

L'enregistrement peut être restitué sous forme graphique.

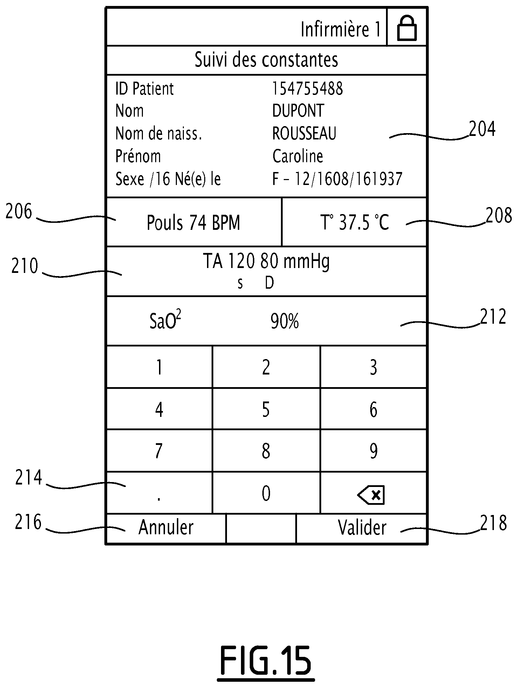

Le menu de suivi des constantes associé est présenté à la figure 15.

Le menu de suivi comprend une pluralité de zones interactives : une zone 204 d'affichage des premières données 190, une zone 206 pour renseigner le pouls du patient 168, une zone 208 pour renseigner la température du patient 168, une zone 210 pour renseigner la tension artérielle du patient 168, une zone 212 pour renseigner la saturation artérielle en oxygène du patient 168, un clavier numérique 214 permettant de renseigner les zones 206 à 212, un bouton d'annulation 216 et un bouton de validation 218 des données renseignées.

En variante, les zones 206 à 212 sont propres à afficher des données du patient 168, les données du patient 168 étant stockées sur une base de données. En particulier, la zone 206 permet de consulter le pouls du patient 168, la zone 208 permet de consulter la température du patient 168, la zone 210 permet de consulter la tension artérielle du patient 168, la zone 212 permet de consulter la saturation artérielle en oxygène du patient 168.

Une deuxième fonctionnalité est d'organiser le travail le suivi de l'administration étant géré par l'opérateur 170.

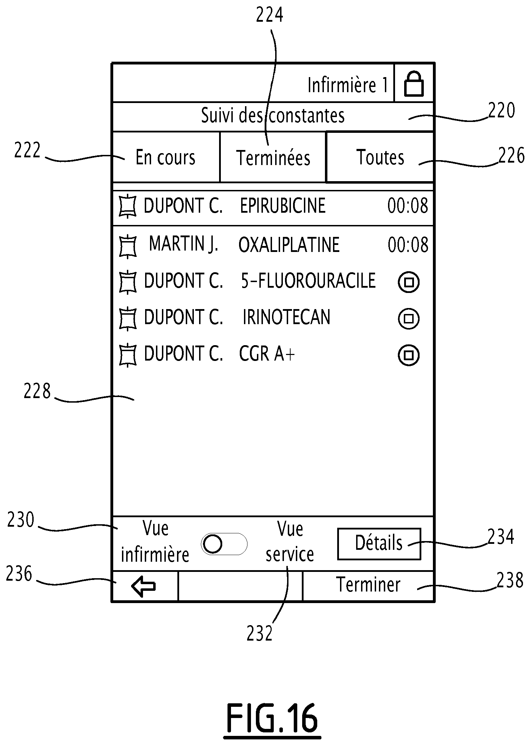

Le menu du tableau de bord est visible à la figure 16.

Le menu du tableau de bord comprend une pluralité de zones interactives : une zone 220 indiquant le titre du menu, un bouton 222 permettant d'afficher l'ensemble des patients 168 dont l'administration du produit est en cours, un bouton 224 permettant d'afficher l'ensemble des patients 168 dont l'administration du produit est terminée, un bouton 226 permettant d'afficher l'ensemble des patients 168, une zone d'affichage 228 des patients 168 sélectionnés aux moyens des boutons 222 à 226 ainsi que le produit administré ainsi que le temps restant.

Le menu du tableau de bord comprend, en outre, un bouton 230 permettant de basculer l'affichage dans la zone 228 entre les patients 168 d'un opérateur 170 donné et l'ensemble des patients 168 traités par le service, un bouton 234 permettant d'afficher les premières données 1490 associé à un patient 18 sélectionné dans la zone d'affichage 228, un bouton 236 de retour au menu précédent et un bouton 238 permettant de fermer le menu.

Une troisième fonctionnalité est de déclencher la facturation. Pour cela, l'assistant personnel 172 comporte un organe de communication interagissant avec un module de facturation.

L'emploi du quatrième ensemble permet de s'assurer que le bon produit est administré au bon patient.

### Conclusion

L'installation permet d'assurer la traçabilité de conteneurs de préparation médicales à chaque instant depuis la préparation jusqu'à l'administration.

L'installation permet d'optimiser et de sécuriser le circuit des préparations de chimiothérapie.

L'installation est de mise en œuvre simple.

L'installation peut s'adapter à tous les types d'établissement ayant une activité en chimiothérapie et même avec l'hospitalisation à domicile.

L'installation procure une traçabilité et une sécurité renforcées.

L'installation permet également d'optimiser la logistique notamment en termes d'efficience ou de gain de temps.

Du point de vue du personnel, cela permet de le soulager de certaines tâches administratives pour qu'il maximise son temps consacré aux patients ainsi que de supprimer les situations de stress dues au risque d'erreur d'administration.

Il en résulte une meilleure maîtrise des coûts.

Cela permet de suivre à tout instant l'avancement des administrations.

Il est à noter que l'installation n'a pas besoin de connexion au réseau informatique de l'établissement.

L'installation ne préjugeant pas du type de produit, l'installation est également compatible avec la transfusion.

## Revendications

1. Terminal mobile de contrôle d'au moins un conteneur (106) comprenant un produit médical destiné à un patient unique, l'au moins un conteneur (106) étant muni d'une étiquette électronique (108) comprenant une mémoire mémorisant des premières données (190) parmi lesquelles au moins une donnée spécifique au produit médical, le terminal mobile comportant :
- un premier lecteur de code-barres lisant des deuxièmes données (192) sur un bracelet d'identification (174) propre à un patient (168),
- un deuxième lecteur lisant les premières données (190), le deuxième lecteur étant un lecteur RFID propre à lire les premières données présentes sur la mémoire de l'étiquette électronique (108),
- un contrôleur comparant les premières données (190) lues par le deuxième lecteur et les deuxièmes données (192) lues par le premier lecteur de code-barres pour obtenir un résultat de comparaison et validant que le produit médical est destiné au patient (168) portant le bracelet d'identification (174) en fonction du résultat de comparaison,
le terminal mobile de contrôle étant également propre à :
- afficher la validation ou l'invalidation par le contrôleur que le produit médical est destiné au patient (168) portant le bracelet d'identification (174),
- afficher le temps restant pour une administration du produit médical, et
- arrêter une administration du produit médical après une ultérieure lecture du conteneur (106) par le deuxième lecteur.

2. Terminal mobile de contrôle selon la revendication 1, dans lequel le terminal mobile de contrôle est un assistant personnel (172).

3. Terminal mobile de contrôle selon la revendication 1 ou 2, dans lequel le terminal mobile comporte une mémoire enregistrant des constantes physiologiques propre au patient (168).

4. Terminal mobile de contrôle selon l'une quelconque des revendications 1 à 3, dans lequel le terminal mobile comporte un organe de communication interagissant avec un module de facturation.

5. Ensemble de contrôle d'au moins un conteneur (106) comprenant un produit médical destiné à un patient (168) unique, l'au moins un conteneur (106) étant muni d'une étiquette électronique (108) comprenant une mémoire mémorisant des premières données (190) parmi lesquelles au moins une donnée spécifique au produit médical, l'ensemble comportant un terminal selon l'une quelconque des revendications 1 à 4.

6. Installation de contrôle d'au moins un conteneur (106) comprenant un produit médical destiné à un patient (168) unique, l'au moins un conteneur (106) étant muni d'une étiquette électronique (108) comprenant une mémoire mémorisant des premières données parmi lesquelles au moins une donnée spécifique au produit médical, l'installation comportant un ensemble selon la revendication 5.

7. Installation selon la revendication 6, l'installation comportant des bornes de contrôle (150) pourvues d'un lecteur lisant les premières données.

8. Installation selon la revendication 6 ou 7, l'installation comportant un dispenseur (100) de contrôle d'au moins un conteneur (106) comprenant un produit médical, l'au moins un conteneur (106) étant muni d'une étiquette électronique (108) comprenant une mémoire mémorisant au moins une information spécifique audit produit médical, le dispenseur (100) comprenant :
- une pluralité de parois internes (111) délimitant un volume de stockage (120), la pluralité de parois internes (111) comprenant des parois latérales (122) et une paroi inférieure (124) sur laquelle les conteneurs (106) ou le colis comprenant les conteneurs (106) sont destinés à reposer, les parois latérales (122) délimitant une unique ouverture d'accès (126) au volume de stockage (120), le dispenseur (100) étant propre à recevoir l'au moins un conteneur (106) ou un colis comprenant l'au moins un conteneur (106) dans le volume stockage ; et
- un lecteur (110) propre à lire l'au moins une information mémorisée dans la mémoire de chaque étiquette électronique (108), le lecteur (110) comprenant une pluralité d'antennes (132) sous forme de boucle, chaque paroi latérale (122) comportant un support (138) propre à loger au moins une partie de l'une des antennes (132).

9. Produit programme d'ordinateur comportant un support lisible d'informations, sur lequel est mémorisé un programme d'ordinateur comprenant des instructions de programme, le programme d'ordinateur étant chargeable sur une unité de traitement de données et adapté pour entraîner la mise en œuvre par le contrôleur du terminal selon l'une quelconque des revendications 1 à 4 d'au moins la comparaison des premières données et des deuxièmes données pour obtenir un résultat de comparaison et la validation du fait que le produit médical est destiné au patient portant le bracelet d'identification en fonction du résultat de comparaison lorsque le programme d'ordinateur est mis en œuvre sur l'unité de traitement des données.

10. Support lisible d'informations sur lequel est mémorisé un produit programme d'ordinateur selon la revendication 9.

## Patentansprüche

1. Mobiles Endgerät zum Steuern mindestens eines Behälters (106), der ein für einen einzelnen Patienten bestimmtes medizinisches Produkt umfasst, wobei der mindestens eine Behälter (106) mit einem elektronischen Etikett (108) versehen ist, das einen Speicher umfasst, der erste Daten (190) speichert, darunter mindestens einen für das medizinische Produkt spezifischen Datenwert, wobei das mobile Endgerät Folgendes aufweist:
- ein erstes Strichcode-Lesegerät, das zweite Daten (192) auf einem Identifikationsarmband (174) eines Patienten (168) liest,
- ein zweites Lesegerät, das die ersten Daten (190) liest, wobei das zweite Lesegerät ein RFID-Lesegerät ist, das geeignet ist, die ersten Daten zu lesen, die im Speicher des elektronischen Etiketts (108) vorhanden sind,
- eine Steuerung, die die ersten Daten (190), die von dem zweiten Lesegerät gelesen wurden, und die zweiten Daten (192), die von dem ersten Strichcode-Lesegerät gelesen wurden, vergleicht, um ein Vergleichsergebnis zu erhalten, und in Abhängigkeit von dem Vergleichsergebnis bestätigt, dass das medizinische Produkt für den Patienten (168) bestimmt ist, der das Identifikationsarmband (174) trägt,
wobei das mobile Steuer-Endgerät ebenfalls geeignet ist:
- die Bestätigung oder Nicht-Bestätigung durch die Steuerung anzuzeigen, dass das medizinische Produkt für den Patienten (168) bestimmt ist, der das Identifikationsarmband (174) trägt,
- die verbleibende Zeit für eine Verabreichung des medizinischen Produkts anzuzeigen, und
- eine Verabreichung des medizinischen Produkts nach einem späteren Lesen des Behälters (106) durch das zweite Lesegerät abzubrechen.

2. Mobiles Steuer-Endgerät nach Anspruch 1, wobei das mobile Steuer-Endgerät ein persönlicher Assistent (172) ist.

3. Mobiles Steuer-Endgerät nach Anspruch 1 oder 2, wobei das mobile Endgerät einen Speicher aufweist, der physiologische Konstanten speichert, die für den Patienten (168) spezifisch sind.

4. Mobiles Steuer-Endgerät nach einem der Ansprüche 1 bis 3, wobei das mobile Endgerät ein Kommunikationselement aufweist, das mit einem Abrechnungsmodul interagiert.

5. Anordnung zum Steuern mindestens eines Behälters (106), der ein für einen einzelnen Patienten (168) bestimmtes medizinisches Produkt umfasst, wobei der mindestens eine Behälter (106) mit einem elektronischen Etikett (108) versehen ist, das einen Speicher umfasst, der die ersten Daten (190) speichert, darunter mindestens einen für das medizinische Produkt spezifischen Datenwert, wobei die Anordnung ein Endgerät nach einem der Ansprüche 1 bis 4 aufweist.

6. Anlage zum Steuern mindestens eines Behälters (106), der ein für einen einzelnen Patienten (168) bestimmtes medizinisches Produkt umfasst, wobei der mindestens eine Behälter (106) mit einem elektronischen Etikett (108) versehen ist, das einen Speicher umfasst, der erste Daten speichert, darunter mindestens einen für das medizinische Produkt spezifischen Datenwert, wobei die Anlage eine Anordnung nach Anspruch 5 aufweist.

7. Anlage nach Anspruch 6, wobei die Anlage Steuerpunkte (150) aufweist, die mit einem Lesegerät versehen sind, das die ersten Daten liest.

8. Anlage nach Anspruch 6 oder 7, wobei die Anlage einen Spender (100) zum Steuern mindestens eines Behälters (106) aufweist, der ein medizinisches Produkt umfasst, wobei der mindestens eine Behälter (106) mit einem elektronischen Etikett (108) versehen ist, das einen Speicher umfasst, der mindestens eine für das medizinische Produkt spezifische Information speichert, wobei der Spender (100) Folgendes umfasst:
- eine Vielzahl von Innenwänden (111), die ein Speichervolumen (120) begrenzen, wobei die Vielzahl von Innenwänden (111) Seitenwände (122) und eine Bodenwand (124) umfasst, auf der die Behälter (106) oder das Paket, das die Behälter (106) umfasst, aufliegen soll, wobei die Seitenwände (122) eine einzelne Zugangsöffnung (126) zu dem Speichervolumen (120) begrenzen, wobei der Spender (100) geeignet ist, den mindestens einen Behälter (106) oder ein Paket, das den mindestens einen Behälter (106) umfasst, in dem Speichervolumen zu empfangen; und
- ein Lesegerät (110), das geeignet ist, mindestens eine Information zu lesen, die im Speicher jedes elektronischen Etiketts (108) gespeichert ist, wobei das Lesegerät (110) eine Vielzahl von Antennen (132) in Schleifenform umfasst, wobei jede Seitenwand (122) einen Träger (138) umfasst, der geeignet ist, mindestens einen Teil einer der Antennen (132) aufzunehmen.

9. Computerprogrammprodukt, das einen lesbaren Informationsträger aufweist, auf dem ein Computerprogramm gespeichert ist, das Programmanweisungen umfasst, wobei das Computerprogramm auf eine Datenverarbeitungseinheit ladbar und zum Training der Durchführung durch die Steuerung des Endgeräts nach einem der Ansprüche 1 bis 4 mindestens des Vergleichs der ersten Daten und der zweiten Daten geeignet ist, um ein Vergleichsergebnis und in Abhängigkeit von dem Vergleichsergebnis die Bestätigung der Tatsache zu erhalten, dass das medizinische Produkt für den Patienten bestimmt ist, der das Identifikationsarmband trägt, wenn das Computerprogramm auf der Datenverarbeitungseinheit ausgeführt wird.

10. Lesbarer Informationsträger, auf dem ein Computerprogrammprodukt nach Anspruch 9 gespeichert ist.

## Claims

1. A mobile controlling terminal for at least one container (106) comprising a medical product intended for a single patient, the at least one container (106) being provided with an electronic tag (108) comprising a memory storing first data (190) among which at least one data item specific to the medical product, the mobile terminal comprising:
- a first barcode reader reading second data (192) on an identification bracelet (174) specific to a patient (168),
- a second reader reading the first data (190), the second reader being an RFID reader suitable for reading the first data (190) within the memory of the electronic tag (108),
- a controller comparing the first data (190) read by the second reader and the second data (192) read by the first barcode reader to obtain a comparison result and validating that the medical product is intended for the patient (168) wearing the identification bracelet (174) based on the comparison result,
the mobile controlling terminal being also suitable for:
- displaying the validation or the invalidation by the controller of whether the medical product is destined to the patient (168) wearing the identification bracelet (174),
- displaying the time remaining before an administration of the medical product, and
- stopping an administration of the medical product after reading again the container (106) by the second reader.

2. The mobile controlling terminal according to claim 1, wherein the mobile device is a personal assistant (172).

3. The mobile controlling terminal according to claim 1 or 2, wherein the mobile terminal has a memory storing physiological vital signs specific to the patient (168).

4. The mobile controlling terminal according to any of claims 1 to 3, wherein the mobile terminal has a communication device interacting with a billing module.

5. An assembly for controlling at least one container (106) comprising a medical product intended for a single patient (168), at least one container (106) being provided with an electronic tag (108) comprising a memory storing first data (190) among which at least one data item specific to the medical product, the assembly comprising a terminal according to any one of claims 1 to 4.

6. An installation for monitoring at least one container (106) comprising a medical product intended for a single patient (168), at least one container (106) being provided with an electronic tag (108) comprising a memory storing first data including at least one data item to the medical product, the installation comprising an assembly according to claim 5.

7. The installation according to claim 6, the installation comprising control terminals (150) provided with a reader that reads the first data.

8. The installation according to claim 6 or 7, the installation comprising a dispenser (100) for checking at least one container (106) containing a medical product, at least one container (106) being provided with an electronic tag (108) comprising a memory storing at least one item of information specific to the said medical product, the dispenser (100) comprising:
- multiple inner walls (111) delimiting a storage volume (120), the multiple inner walls (111) comprising side walls (122) and a bottom wall (124) on which the containers (106) or the package comprising the containers (106) are intended to rest, the side walls (122) delimiting a single access opening (126) to the storage volume (120), the dispenser (100) being adapted to receive at least one container (106) or a package comprising at least one container (106) in the storage volume; and
- a reader (110) suitable for reading at least one item of information stored in the memory of each electronic tag (108), the reader (110) comprising multiple antennas (132) in the form of a loop, each side wall (122) comprising a support (138) able to house at least part of one of the antennas (132).

9. A computer program product having a readable information medium on which a computer program including program instructions is stored, the computer program being loadable on a data processing unit and adapted to cause the terminal controller to implement according to any of claims 1 to 4 at least the comparison of the first data and the second data to obtain a comparison result and the validation that the medical product is intended for the patient wearing the identification bracelet based on the comparison result when the computer program is implemented on the data processing unit.

10. A readable information carrier on which computer software according to claim 9 is stored.
